# EUROPÄISCHE PATENTSCHRIFT

(11) **EP 0 499 873 B1**
(45) Veröffentlichungstag und Bekanntmachung des Hinweises auf die Patenterteilung: **22.11.2000**
(21) Anmeldenummer: 92101809.9
(22) Anmeldetag: 04.02.1992
(51) Int. Cl.: C07D 207/16, C07D 207/08, C07D 207/09, C08G 18/20, C07D 487/04, C07D 209/54, C07D 403/06

(54) **Verwendung von 3,(4)-substituierten Pyrrolidinen als Katalysatoren für das Polyisocyanatpolyadditionsverfahren sowie Verfahren zur Herstellung von Polyurethan(harnstoff)en**
Use of 3,(4)-substituted pyrrozidines as catalysts in the polyisocyanate-polyaddition process and a process for the preparation of polyurethanes and polyureas
Utilisation de pyrrolidines 3(4)-substituées comme catalyseurs pour le procédé de polyaddition de polyisocyanates et procédé pour la fabrication de polyurethanes et polyurées

(30) Priorität: 17.02.1991 DE 4104870
(43) Veröffentlichungstag der Anmeldung: 26.08.1992
(60) Teilanmeldung: 96106083.7
(73) Patentinhaber: BAYER AG, 51368 Leverkusen (DE)
(72) Erfinder: Weider, Richard, Dr., W-5090 Leverkusen 3 (DE); Scholz, Uwe, Dr., W-5000 Köln 80 (DE); Ruckes, Andreas, Dr., W-5090 Leverkusen (DE)

(56) Entgegenhaltungen:
- EP-A- 0 255 908
- EP-A- 0 296 560
- EP-A- 0 393 607
- DE-A- 2 116 535
- US-A- 3 318 908
- US-A- 3 544 590
- US-A- 4 563 484
- J. HETEROCYCLIC CHEMISTRY Bd. 25, Nr. 6, 1988, Seiten 1665 - 73; F. ORSINI ET AL.: '1,3-Dipolar Cycloadditions of Azomethine Ylides with Dipolarophiles. II. Synthesis of Pyrrolizidines' gesamtes Dokument
- GAZZ. CHIM. ITAL. Bd. 115, Nr. 10, 1985, Seiten 569 - 71; M. FORTE ET AL.: 'Synthesis of pyrrolizidines and 1-oxapyrrolizidines from proline' gesamtes Dokument
- CHEMICAL ABSTRACTS, vol. 71, no. 13, 29. September 1969, Columbus, Ohio, US; abstract no. 61109E, S. OHKI ET AL.: 'Synthesis of pyrrolidine derivatives with pharmacological activity. V. Synthesis of alpha-cycloalkyl-alpha-phenyl-3-pyr rolidinomethanol derivatives' Seite 419 ;
- CHEMICAL ABSTRACTS, vol. 101, 10. September 1984, Columbus, Ohio, US; abstract no. 90743A, S. BINIECKI, J. KRSZEMINSKI: 'Synthesis of indolizidine derivatives' Seite 624 ;
- PATENT ABSTRACTS OF JAPAN
- HELV. CHIM. ACTA Bd. 64, Nr. 7, 1981, Seiten 2203 - 18; R. ACHINI: 'Synthesis of Phenyl- and Benzyl-Substituted Pyrrolidines and of a Piperidine by Intramolecular C-Alkylation. Synthons for Tricyclic Skeletons' gesamtes Dokument
- HETEROCYCLES Bd. 23, Nr. 3, 1985, Seiten 653 - 9; J. CHASTANET, G. ROUSSI: 'A new route to hexahydropyrrolizine derivatives via nonstabilized ylide generated from N-methylpyrrolidone N-oxide' gesamtes Dokument
- J. ORG. CHEM. Bd. 50, Nr. 16, 1985, Seiten 2910 - 14; J. CHASTANET, G. ROUSSI: 'N-Methylpiperidine N-Oxide as a Source of Nonstabilized Ylide: A New and Efficient Route to Octahydroindolizidine Derivatives' gesamtes Dokument
- SYNTHESIS 1980, Seiten 811 - 12; T. ASAI ET AL.: 'New Methods and Reagents in Organic Synthesis; 7. alpha-Alkylation of Benzylamine under Phase-Transfer Catalyzed Conditions' Verbindung der Formel 4

## Beschreibung

Die Erfindung betrifft die Verwendung von 3- und/oder 4-substituierten Pyrrolidinen als Katalysatoren zur Herstellung von Produkten nach dem Polyisocyanatpolyadditionsverfahren. Sie können als Ersatz oder in Kombination mit an sich bekannten Urethankatalysatoren z.B. 1,4-Diazobicyclo[2,2,2]octan (DABCO) zur Herstellung von starren oder flexiblen Polyurethanschäumen sowie zahlreichen anderen Polyurethan-Produkten eingesetzt werden. Unter dem obengenannten Begriff "Polyurethan-Produkte" sollen im folgenden alle Umsetzungsprodukte von Polyisocyanaten mit Verbindungen mit mindestens 2 gegenüber Isocyanaten reaktionsfähigen Wasserstoffatomen gemeint sein, d.h. unter dem hier verwendeten Begriff Polyurethan sind beispielsweise reine Polyurethane, Polyurethan-polyharnstoffe oder reine Polyharnstoffe zu versehen.

Die Geschwindigkeit der Reaktion zwischen Isocyanat-Gruppen und Verbindungen mit gegenüber NCO-Gruppen reaktionsfähigen Wasserstoffatomen wird außer von der Temperatur der Ausgangsprodukte und ihrer Struktur vor allem durch geeignete Katalysatoren beeinflußt. In der Praxis werden als nucleophile Katalysatoren hauptsächlich Basen (z.B. tertiäre Amine wie Triethylamin) und als elektrophile Katalysatoren organische Metallverbindungen (z.B. Sn-Carboxylate wie Sn(II)-octoat) eingesetzt. Stand der Technik ist die gemeinsame Verwendung von Lewis-Säuren und Lewis-Basen, die üblicherweise durch synergistische Effekte gekennzeichnet ist. Es ist jedoch auch bekannt, daß in einer Vielzahl von Anwendungen ausschließlich Amine als Katalysatoren eingesetzt werden. Von der großen Anzahl bekannter Amin-Katalysatoren (vgl. Ullmann 4. Auflage und Kunststoffhandbuch Band VII, Polyurethane, Hansen-Verlag, München 1983) haben bisher jedoch nur relativ wenige auf breiter Basis Eingang in die Technik gewonnen, wie z.B. 1,4-Diazabicyclo[2,2,2]octan, Bis-(2-dimethylaminoethyl)ether, Triethylamin, Dimethylcyclohexylamin, Dimethylethanolamin, Dimethylbenzylamin, Methylmorpholin, Ethylmorpholin, um die wichtigsten zu nennen. Insbesondere werden naturgemäß solche Katalysatoren verwendet, die sich durch hohe Aktivität, wirtschaftliche Herstellbarkeit sowie breite Aufwendbarkeit auszeichnen. Ein weiterer ständig an Bedeutung zunehmender Aspekt ist die Bewertung der Katalysatoren unter toxikologischen Gesichtspunkten hinsichtlich Verarbeitungssicherheit und Geruchsbelästigung. Viele der heute verwendeten Amin-Katalysatoren, wie z.B. DABCO oder Triethylamin, sind aus dieser Sicht aufgrund ihrer hohen Flüchtigkeit und ihres relativ intensiven Amingeruchs, welcher in das endgültige, damit hergestellte Produkt übertragen wird, als unbefriedigend zu bezeichnen. Aufgrund der Vielzahl von Anwendungsmöglichkeiten von Polyurethan-Kunststoffen ist es ebenso wünschenswert, den jeweiligen Bedürfnissen angepaßte, d.h. "maßgeschneiderte" Katalysatoren zur Verfügung zu stellen. Eine Möglichkeit ist die chemische Modifizierung eines Katalysator-Typs, um dessen Aktivität auf den jeweiligen Verwendungszweck abzustimmen.

Überraschenderweise wurde nun gefunden, daß bestimmte Pyrrolidinderivate mit Vorteil als Katalysatoren zur Herstellung von Polyurethanen verwendet werden können. Die erfindungsgemäßen Verbindungen besitzen im Vergleich zu den oben erwähnten aminischen Katalysatoren eine vergleichbare oder noch höhere Aktivität. Ein anderer willkommener Effekt ist bei den erfindungsgemäß vorgeschlagenen Katalysatoren darin zu sehen, daß sich durch Wahl geeigneter Substituenten am Ring die Aktivität der Produkte - z.B. im Gegensatz zum DABCO, das unter wirtschaftlich vertretbaren Aspekten keine Variation erlaubt, - maßgeschneidert einstellen läßt. Vorteilhaft ist weiterhin der geringe Eigengeruch sowie die geringe Flüchtigkeit der Verbindungen, die z.T. über isocyanatreaktive Gruppen in das Polymer eingebunden werden, die zu einer deutlich verminderten Geruchsbelästigung bei der Herstellung von Polyurethan-Produkten führt. Zusätzlich können weitere Vorteile beobachtet werden, wie z.B. leichte Handhabung, da die bevorzugt eingesetzten Pyrrolidine flüssig sind, ein guter Härtungsverlauf sowie nicht zuletzt die teilweise sehr einfache Herstellung der Verbindungen.

Gegenstand der vorliegenden Erfindung ist somit die Verwendung von 3,(4)-substituierten Pyrrolidinen gemäß der nachfolgenden Formel (I) 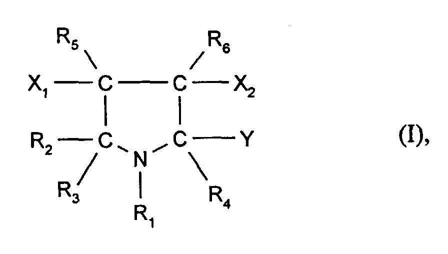 wobei
- R₁: Wasserstoff, eine C₁-C₁₂-Alkyl-, C₃-C₇-Cycloalkyl-, C₆-C₁₄-Aryl- oder -Arylalkyl-Gruppe, die gegebenenfalls jeweils N oder O enthalten können, oder gemeinsam mit R₄ eine C₁-C₅-Alkylengruppe oder eine C₁-C₆-Alkylengruppe, die zwei Pyrrolidinreste gemäß Formel (I) über das N-Atom verbindet;
- R₂, R₃: unabhängig voneinander Wasserstoff, eine C₁-C₁₂-Alkyl-, C₃-C₇-Cycloalkyl-, -Aryl- oder C₆-C₁₄-Arylalkylgruppe oder gemeinsam eine C₂-C₉-Alkylengruppe;
- R₄: Wasserstoff, eine C₁-C₁₂-Alkyl-, C₃-C₇-Cycloalkyl-, C₆-C₁₄-Aryl- oder -Arylalkyl-Gruppe oder gemeinsam mit R₁ eine C₁-C₅-Alkylengruppe;
- R₅, R₆: Gruppen mit der gleichen Bedeutung wie Definition für R₂, R₃ oder gemeinsam einen Rest der Struktur: 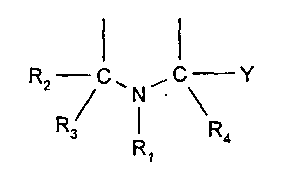
- X₁, X₂: unabhängig voneinander (d.h. gleich oder verschieden) Wasserstoff, Carbonsäuregruppen oder deren Alkylester, die gegebenenfalls O- oder N-Atome enthalten können, oder Nitril oder die Gruppen -CHR₇OH, -CH₂NR₈R₉, -CONR₈R₉, -NR₈R₉ oder -CONR₈-(CH₂)₁₋₆-NR₇R₈ wobei
- R₇: Wasserstoff, eine C₁-C₁₂-Alkyl-, -Cycloalkyl-, -Aryl-, -Arylalkylgruppe bedeutet und
- R₈, R₉: die Bedeutung wie Definition für R₂, R₃ besitzen;
- Y: Wasserstoff oder eine Carbonsäuregruppe oder deren Alkylester, die gegebenenfalls O- oder N-Atome enthalten können, oder Nitril oder die Gruppe -CHR₇OH oder -CH₂NR₈R₉;
darstellen, wobei mindestens einer der Reste X₁ oder X₂ von Wasserstoff verschieden sein muß und es sich im Fall X₁ ungleich X₂, um Gemische verschiedener Isomere bezüglich der Stellung der Substituenten X₁ und X₂ in der 3- oder 4-Position des Pyrrolidinrings, verschiedener Stereoisomere oder um isomerenreine Verbindungen handeln kann,
als Katalysatoren für das Polyisocyanatpolyadditionsverfahren.

Bevorzugte Verbindungen sind dabei Katalysatoren der allgemeinen Formel (Ia) 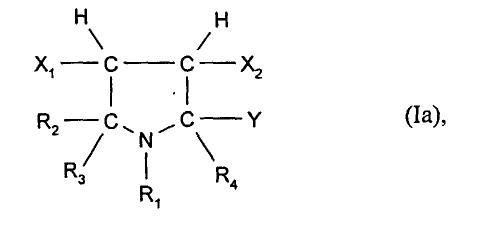 wobei
- R₁ bis R₄: die Bedeutung aus Formel (I) haben,
- R₅ und R₆: Wasserstoff;
- X₁, X₂: Wasserstoff oder Nitril oder die Gruppen -CH₂OH, -CH₂NH₂ oder -NH₂ oder Carbonsäureester oder -amide, die gegebenenfalls O- oder N-Atomen enthalten,
und
- Y: Wasserstoff oder -CH₂OH oder -CH₂NH₂
darstellen.

Besonders bevorzugt werden Katalysatoren der allgemeinen Formel (Ib) 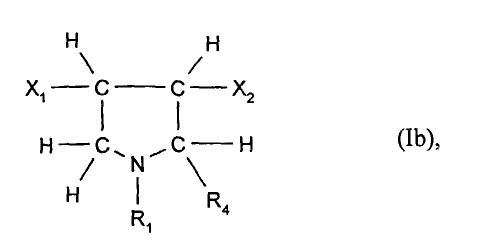 wobei
- X₁ und X₂: die Bedeutung gemäß Formel (Ia) haben und
- R₁: eine C₁-C₁₂-Alkyl, C₅-C₆-Cycloalkyl-, Aryl-, Arylalkyl-Gruppe oder gemeinsam mit R₄ eine C₃-C₅-Alkylengruppe;
- R₂, R₃: und Y Wasserstoff;
und
- R₄: Wasserstoff oder gemeinsam mit R₁ eine C₃-C₅-Alkylengruppe
darstellen , verwendet.

Die Herstellung der erfindungsgemäßen Pyrrolidine erfolgt aus gut verfügbaren und preiswerten Rohstoffen wie Aminosäurederivaten, Ketonen oder Aldehyden und Olefinen in sehr einfacher Weise und ist in den wesentlichen Stufen z.B. in Bull. Soc. Chim. France, 1988, S. 579-583 oder in Bull. Chem. Soc. Japan, 60, S. 4.079-4.090 (1989) beschrieben. Das dort beschriebene Verfahren erfordert allerdings zur Erzielung guter Ausbeuten sehr geringe Konzentrationen (unter 0,1 mol/l, vorteilhaft ca. 0,04 mol/l (s. Bull. Soc. Chim. France, S. 581 r.oben)) und damit für eine wirtschaftliche Herstellung nicht mehr akzeptable Lösungsmittelmengen. Führt man das beschriebene Verfahren bei höheren Konzentrationen (z.B. 1 mol/l) durch, sinken die Ausbeuten an gewünschten Verbindungen drastisch auf unter 30% zugunsten unlöslicher, nicht destillierbarer, inaktiver Harze. Selbst bei einer höheren Verdünnung zwischen 0,1 und 0,2 mol/l bewegen sich die Ausbeuten nicht in einer wirtschaftlich vertretbaren Größenordnung. Eine Verwendung der substituierten Pyrrolidine in technischem Maßstab ist nach diesem Verfahren nicht möglich.

Es wurde nun gefunden, daß man auch bei wesentlich höheren und damit wirtschaftlichen Konzentrationen zu sehr hohen Ausbeuten gelangt, wenn man die Keton- oder Aldehydkomponente während des Reaktionsverlaufs langsam zudosiert. Das Fortschreiten der Reaktion und damit die Zugabegeschwindigkeit ist leicht durch Gaschromatographie oder durch Bestimmung der Menge des gebildeten Reaktionswassers festzustellen. Ein solches Herstellungsverfahren ist in der Teilanmeldung (Anmeldungs-Nr. 96 106083.7) beschrieben.

Bevorzugte erfindungsgemäße Pyrrolidine sind z.B.:
3-Hydroxymethyl-N-methyl-pyrrolidin, 3,4-Bis-hydroxymethyl-N-methyl-pyrrolidin, 3-Aminomethyl-N-methyl-pyrrolidin, N-Methyl-pyrrolidin-3-carbonsäure-N-(3-dimethylaminopropyl)-amid, 1-Azabicyclo[3,3,0]-octan-3-carbonsäurebutylester, 1-Azabicyclo[3,3,0]-octan-4-carbonsäurebutylester, 1-Azabicyclo[4,3,0]-nonan-3-carbonsäurebutylester, 1-Azabicyclo[4,3,0]-nonan-4-carbonsäurebutylester, 3-Cyano-1-azabicyclo[3,3,0]-octan, 4-Cyano-1-azabicyclo[3,3,0]-octan, 3-Aminomethyl-1-azabicyclo[3,3,0]-octan und 4-Aminomethyl-1-azabicyclo[3,3,0]-octan.

Besonders bevorzugt sind 3-Hydroxymethyl-N-methyl-pyrrolidin, 3,4-Bis-hydroxymethyl-N-methyl-pyrrolidin, 3-Aminomethyl-N-methyl-pyrrolidin, N-Methyl-pyrrolidin-3-carbonsäure-N-(3-dimethylaminopropyl)-amid, 1-Azabicyclo[3,3,0]-octan-3-carbonsäurebutylester, 1-Azabicyclo[3,3,0]-octan-4-carbonsäurebutylester,3-Aminomethyl-1-azabicyclo[3,3,0]-octan und 4-Aminomethyl-1-azabicyclo[3,3,0]-octan.

Die neuen erfindungsgemäßen Katalysatoren sind farblose bis leicht gelbliche Verbindungen, wobei die bevorzugten Typen flüssig sind. Sie sind löslich in organischen Lösungsmitteln und löslich oder dispergierbar in Wasser.

Gegenstand der Erfindung ist auch das Verfahren zur Herstellung von gegebenenfalls zellförmigen Kunststoffen nach dem Polyisocyanatpolyadditionsverfahren durch Umsetzung von
a) Polyisocyanaten mit
b) höhermolekularen Verbindungen mit mindestens zwei gegenüber Isocyanaten reaktionsfähigen Wasserstoffatomen, unter Verwendung von
c) Katalysatoren
   sowie gegebenenfalls
d) Kettenverlängerungsmitteln und
e) weiteren an sich bekannten Zusatzmitteln wie Treibmitteln, Stabilisatoren und andere,
das dadurch gekennzeichnet ist, daß als Katalysatoren solche der allgemeinen Formel (I), gegebenenfalls in Kombination mit herkömmlichen in der Polyurethanchemie üblichen Katalysatoren, eingesetzt werden.

Erfindungsgemäß bevorzugt soll der Anteil der Katalysatoren nach Formel (I) mindestens 50 Gew.-% der insgesamt eingesetzten Katalysatoren betragen.

Die neuen Katalysatoren werden erfindungsgemäß bevorzugt in Mengen von 0,01 bis 5 Gew.-%, bezogen auf die Verbindung mit den aktiven Wasserstoffatomen, eingesetzt. Es kann zwar mehr als die obengenannte Menge eingesetzt werden, was jedoch keinen Vorteil bringt.

Die aktive Wasserstoffatome besitzenden Verbindungen, welche beim erfindungsgemäßen Verfahren als Komponente b) verwendet werden, sind solche, welche bei den früher bekannten Verfahren zur Polyurethan-Herstellung verwendet worden sind und wie sie z.B. im Kunststoffhandbuch Band VII, Polyurethane, Hansen-Verlag, München 1983 oder im Houben-Weyl, Makromolekulare Stoffe, Band E20, beschrieben werden. Sie besitzen ein Molekulargewicht von 300 bis 10.000, vorzugsweise 1.000 bis 6.000.

Die erfindungsgemäß verwendeten Verbindungen mit NCO-Gruppen, Komponente a), sind die gleichen Verbindungen, welche bei bisher bekannten Verfahren eingesetzt wurden und wie sie z.B. im Kunststoffhandbuch Band VII, Polyurethane, Hansen-Verlag, München 1983 oder im Houben-Weyl, Makromolekulare Stoffe, Band E20, beschrieben werden.

Bei dem erfindungsgemäßen Verfahren werden die substituierten Pyrrolidine in der gleichen Art verwendet, wie man die früheren Katalysatoren eingesetzt hat, z.B. kann der Katalysator in seiner flüssigen Form für sich verwendet werden, oder durch Auflösen in einem Polyol oder einem passenden Lösungsmittel; er kann unter jeglichen Temperatur- oder anderen Bedingungen - entweder allein oder in Kombination anderer in der Technik bekannter Katalysatoren, welche als brauchbar zur Herstellung von Polyurethanen bekannt sind eingesetzt werden: Beispielsweise organischen oder anorganischen Zinnverbindungen oder anderen metallorganischen Verbindungen; tertiären Aminen; Alkanolaminen; cyclischen Aminen; Polyaminen und dergleichen; Alkalimetallverbindungen und anderer Co-Katalysatoren.

Das erfindungsgemäße Verfahren ist für die herkömmlichen Herstellungsmethoden, wie z.B. ONE-SHOT- oder Prepolymerverfahren zur Herstellung von Polyurethanschäumen, Polyurethanelastomeren, Polyurethanüberzügen und dergleichen geeignet, ebenso wie für die Vemetzungsreaktion, welche oft im Anschluß an die direkte Polyaddition erwünscht ist.

Alle anderen Bedingungen sind die gleichen wie bei herkömmlichen Urethanpolyadditionsverfahren. In jedem dieser Fälle können weitere Zusatzmittel, wie Kettenverlängerungsmittel, Treibmittel, Schaumstabilisatoren, Emulgatoren, Farben, Pigmente und Füllstoffe der an sich bekannten Art mitverwendet werden.

Die oben erwähnten erfindungsgemäßen Katalysatoren beschleunigen die Polyaddition in beträchtlichem Ausmaß und demnach ist die benötigte Katalysatormenge sehr gering. Da die erfindungsgemäßen Verbindungen nur einen geringen Geruch besitzen und das sie nicht flüchtige Flüssigkeiten, Feststoffe oder einbaubare Verbindungen darstellen, sind die erhaltenen Polyurethan-Produkte frei von unerwünschten Gerüchen.

Die nachfolgenden Beispiele erläutern die Erfindung weiter, ohne sie zu beschränken. Teile und Verhältnisse sind in allen Fällen als Gewichtsteile gemeint.

### Beispiele

### Beispiel 1

In diesem Beispiel wird das Verfahren zur Herstellung des 3-Cyano-N-methylpyrrolidins und des 3-Aminomethyl-N-methylpyrrolidins beschrieben:
a) Vergleichsbeispiel (nicht erfindungsgemäßes Verfahren):
   In einem 4 l-Dreihalskolben, ausgerüstet mit Rührer, Rückflußkühler und einem ca. 70 ml fassenden Wasserabscheider werden 300 g N-Methylglycin, 300 g Acrylnitril, 105 g Paraformaldehyd und 3 l Toluol vorgelegt und bei einer Badtemperatur von 120 bis 140°C zum kräftigen Rückfluß erhitzt, bis die Wasserentwicklung abgeschlossen ist. Am Ende der Reaktion erhält man eine braungelbe Lösung, aus der sich ein dunkelbraunes Harz abgeschieden hat. Das Lösemittel wird abdestilliert und der Rückstand im Vakuum fraktioniert. Man erhält 96 g (26 % der Theorie) 3-Cyano-N-methylpyrrolidin.
b) Vergleichsbeispiel (nicht erfindungsgemäßes Verfahren):
   In einem 4 l-Dreihalskolben, ausgerüstet mit Rührer, Rückflußkühler und einem ca. 70 ml fassenden Wasserabscheider werden 50 g N-Methylglycin, 60 g Acrylnitril, 25 g Paraformaldehyd und 3 l Toluol vorgelegt und bei einer Badtemperatur von 120 bis 140°C zum kräftigen Rückfluß erhitzt, bis die Wasserentwicklung abgeschlossen ist. Am Ende der Reaktion erhält man eine braungelbe Lösung. Das Lösemittel wird abdestilliert und der Rückstand im Vakuum fraktioniert. Man erhält 32 g (51 % der Theorie) 3-Cyano-N-methylpyrrolidin.
c) Erfindungsgemäßes Verfahren:
   In einem 4 l-Dreihalskolben, ausgerüstet mit Rührer, Rückflußkühler, Dosiertrichter und einem ca. 70 ml fassenden Wasserabscheider werden 300 g N-Methylglycin, 300 g Acrylnitril und 3 l Toluol vorgelegt und bei einer Badtemperatur von 120 bis 140°C zum kräftigen Rückfluß erhitzt. Dann werden in Portionen von 3 g insgesamt 105 g Paraformaldehyd zudosiert, wobei jeweils solange mit der Zugabe der nächsten Portion gewartet wird, bis die Wasserentwicklung abgeschlossen ist. Am Ende der Reaktion erhält man eine homogene, leicht gelbliche Lösung. Das Lösemittel wird abdestilliert und der Rückstand im Vakuum fraktioniert. Man erhält 315 g (85 % der Theorie) 3-Cyano-N-methylpyrrolidin (Kp.: 83-85°C, 22 mmHg).

Die anschließende Reduktion zum 3-Aminomethyl-N-methylpyrrolidin erfolgt nach bekanntem Verfahren:

Man löst die Cyanverbindung im gleichen Volumen Methanol, füllt die Lösung in einen 2 l-Autoklaven, gibt 20 g Raney-Kobalt dazu und drückt 130 g Ammoniak auf. Dann wird bei einem Wasserstoffdruck von 90 bis 100 bar bei 90°C hydriert (ca. 3 Stunden). Die entspannte Lösung wird filtriert, eingedampft und der Rückstand im Vakuum fraktioniert. Man erhält 310 g (95 % der Theorie) 3-Aminomethyl-N-methylpyrrolidin (Kp.: 61°C, 22 mbar).

### Beispiele 2 bis 9

Nach dem in Beispiel 1 c) beschriebenen Verfahren werden unter den gleichen Bedingungen die im Anhang formelmäßig angegebenen Verbindungen hergestellt. Tabelle 1 gibt die Ausgangsstoffe sowie die erzielten Ausbeuten (% der Theorie, bezogen auf Aminosäurederivat) nach destillativer Reinigung an:

**Tabelle 1**

| Beispiel | Aminosäurederivat | Keton/Aldehyd | Olefin | Ausbeute |
|---|---|---|---|---|
| 2 | Methylglycin | Butyraldehyd | Acrylnitril | 87 % |
| 3 | Methylglycin | Aceton | Methylacrylat | 79 % |
| 4 | Methylglycin | Paraformaldehyd | Butylacrylat | 89 % |
| 5 | Methylglycin | Paraformaldehyd | Methylacrylat | 81 % |
| 6 | Methylglycin | Paraformaldehyd | Diethylmaleat | 85 % |
| 7 | DL-Prolin | Paraformaldehyd | Butylacrylat | 95 % |
| 8 | DL-Prolin | Paraformaldehyd | Acrylnitril | 92 % |
| 9 | DL-Prolin | Paraformaldehyd | Diethylmaleat | 90 % |

### Physikalische Daten der Verbindungen 2 bis 9:

- 2:: Kp.: 103-110°C (20 mbar);
- 3:: (2 Isomere) Kp.: 75-78°C (11 mbar);
- 4:: Kp.: 105°C(12 mbar);
- 5:: Kp.: 71°C (12 mbar);
- 6:: (2 Isomere) Kp.: 140-145°C (11 mbar);
- 7:: (4 Isomere) Kp.: 140-147°C (12 mbar);
- 8:: (4 Isomere) Kp.: 115-120°C (22 mbar);
- 9:: (6 Isomere) Kp.: 175-185°C (10 mbar).

### Beispiele 10 bis 14

Die Verbindungen 2 und 8 werden wie in Beispiel 1a) zu den Verbindungen 10 und 11 hydriert, die Verbindungen 5 und 6 werden durch Reduktion mit Natriumborhydrid nach bekanntem Verfahren zu den Verbindungen 12 und 13 reduziert. Die Verbindung 5 wird durch Aminolyse mit 1-Amino-3-dimethylamino-propan nach bekanntem Verfahren zur Verbindung 14 umgesetzt. Die Strukturen sind dem Anhang zu entnehmen.

### Beispiel 15

In diesem Beispiel wird die hohe katalytische Aktivität zweier Vertreter aus der Reihe der 3-substituierten Pyrrolidine in einem Weichschaumsystem gezeigt.

Eingesetzt werden ein 1:1-Gemisch aus 1-Azabicyclo[3,3,0]octan-3- und -4-carbonsäurebutylester (Katalysator 1) sowie 3-Aminomethyl-N-methylpyrrolidin (Katalysator 2), hergestellt nach dem im Text beschriebenen Verfahren.

### A-Komponente:

18 Teile einer Mischung aus 2,4-Toluylendiisocyanat und 2,6-Toluylendiisocyanat im Verhältnis 80:20.

### B-Komponente:

50,00 Teile eines Polyetherpolyols der OH-Zahl 35 mg KOH/g, hergestellt durch Umsetzung von Trimethylolpropan mit Propylenoxid (PO) und anschließender Umsetzung mit Ethylenoxid (EO) mit einem PO/EO-Verhältnis von 86,55/13,45

### ferner:

- 1,50 Teile: Wasser
- 0,50 Teile: eines Polyetherpolysiloxans als Stabilisator; Stabilisator OS 50 der Fa. Bayer AG
- 0,30 Teile: des jeweiligen oben beschriebenen Pyrrolidins.

Die A-Komponente wird mit der B-Komponente vereinigt und mit einem Schnellrührer 10 sec gründlich vermischt. Anschließend wird das Reaktionsgemisch bei Raumtemperatur in einer offenen Form verschäumt.

| | Startzeit | Steigzeit |
|---|---|---|
| Katalysator 1 | 4 sec | 90 sec |
| Katalysator 2 | 4 sec | 90 sec |

### Beispiele 16 bis 20

In diesen Beispielen wird die abstutbare hohe katalytische Aktivität weiterer Vertreter aus der Reihe der 3-substituierten Pyrrolidine in einem PUR-Kaltweichformschaumsystem gezeigt.

Dabei wurden folgende Katalysatoren verwendet:
Katalysator 1: siehe Beispiel 15
Katalysator 2: siehe Beispiel 15
Katalysator 3: N-Methylpyrrolidin-3-carbonsäurebutylester
Katalysator 4: N-Methylpyrrolidin-3-carbonsäure-N(3-dimethylaminopropyl)-amid
alle hergestellt nach dem im Text beschriebenen Verfahren.

### A-Komponente:

37,10 Teile eines Gemisches aus 80 Gew.-% 2,4-Toluylendiisocyanat und 2,6-Toluylendiisocyanat (im Verhältnis 80:20) und 20 Gew.-% 4,4'-Diisocyanatodiphenylmethan mit polymeren Anteilen mit einem NCO-Gehalt von 44,5 ± 0,5 Gew.-%; Handelsprodukt Desmodur® VT 06 der Fa. Bayer AG.

### B-Komponente:

100,00 Teile eines Polyetherpolyols der OH-Zahl 28 ± 2 mg KOH/g, hergestellt durch Umsetzung von Trimethylolpropan (TMP) mit Propylenoxid (PO) und anschließende Umsetzung mit Ethylenoxid (EO) mit einem PO/EO-Verhältnis von 82/18

### ferner:

- 3,00 Teile: Wasser
- 0,05 Teile: einer 70%igen Lösung von Bis-(2-dimethylaminoethyl)ether in Dipropylenglykol (DPG)
- 0,25 Teile: einer 33%igen Lösung von Diazabicyclo[2,2,2]octan (DABCO) in DPG
- 0,20 Teile: Schaumstabilisator B4617 der Fa. Goldschmidt AG
- 0,80 Teile: eines Polyetherpolysiloxans als Stabilisator; Stabilisator OS50 der Fa. Bayer AG
- 0,6 Teile: des betreffenden Katalysators 1 bis 4.
Die Verarbeitung erfolgt wie in Beispiel 15 beschrieben.

In Tabelle 2 sind die Ergebnisse bei der Verwendung der verschiedenen Katalysatoren zusammengefaßt.

**Tabelle 2**

| Beispiel | Katalysator | Startzeit [sec] | Abbindezeit [sec] | Steigzeit [sec] |
|---|---|---|---|---|
| 16 | ohn | 9 | 108 | 213 |
| 17 | 1 | 5 | 46 | 93 |
| 18 | 2 | 4 | 38 | 72 |
| 19 | 3 | 6 | 83 | 120 |
| 20 | 4 | 5 | 61 | 113 |

Man erhält Schäume mit einwandfreier Schaumstruktur.

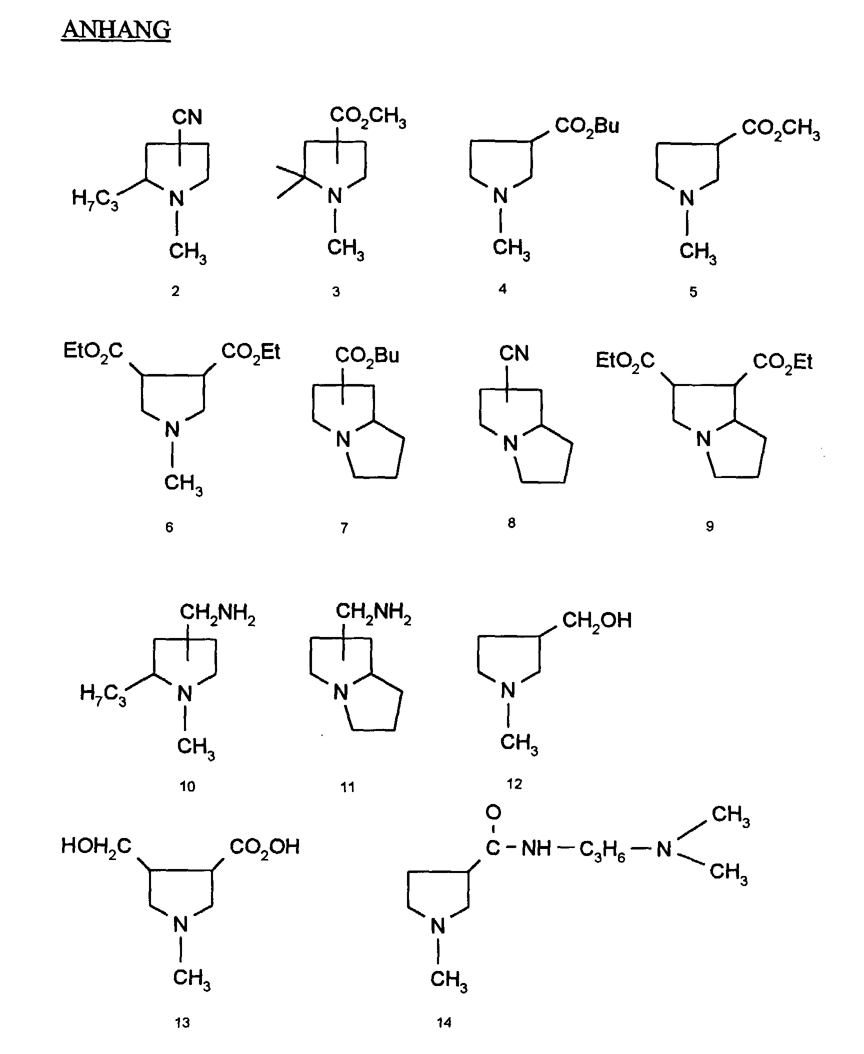

## Patentansprüche

1. Verwendung von 3,(4)-substituierten Pyrrolidinen gemäß der nachfolgenden Formel (I) als Katalysatoren für das Polyisocyanatpolyadditionsverfahren: 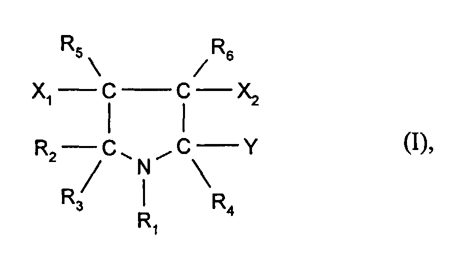 wobei
R₁ Wasserstoff, eine C₁-C₁₂-Alkyl-, C₃-C₇-Cycloalkyl-, C₆-C₁₄-Aryl- oder -Arylalkyl-Gruppe, die gegebenenfalls jeweils N oder O enthalten können, oder gemeinsam mit R₄ eine C₁-C₅-Alkylengruppe oder eine C₁-C₆-Alkylengruppe, die zwei Pyrrolidinreste gemäß Formel (I) über das N-Atom verbindet;
R₂, R₃ unabhängig voneinander Wasserstoff, eine C₁-C₁₂-Alkyl-, C₃-C₇-Cycloalkyl-, -Aryl- oder C₆-C₁₄-Arylalkylgruppe oder gemeinsam eine C₂-C₉-Alkylengruppe;
R₄ Wasserstoff, eine C₁-C₁₂-Alkyl-, C₃-C₇-Cycloalkyl-, C₆-C₁₄-Aryl- oder -Arylalkyl-Gruppe oder gemeinsam mit R₁ eine C₁-C₅-Alkylengruppe;
R₅, R₆ Gruppen mit der gleichen Bedeutung wie Definition für R₂, R₃ oder gemeinsam einen Rest der Struktur: 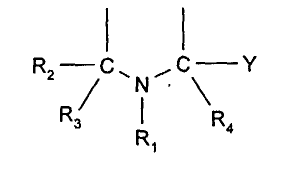
X₁, X₂ unabhängig voneinander (d.h. gleich oder verschieden) Wasserstoff, Carbonsäuregruppen oder deren Alkylester, die gegebenenfalls O- oder N-Atome enthalten können, oder Nitril oder die Gruppen -CHR₇OH, -CH₂NR₈R₉, -CONR₈R₉, -NR₈R₉ oder -CONR₈-(CH₂)₁₋₆-NR₇R₈ wobei
R₇ Wasserstoff, eine C₁-C₁₂-Alkyl-, -Cycloalkyl-, -Aryl-, -Arylalkylgruppe bedeutet und
R₈, R₉ die Bedeutung wie Definition für R₂, R₃ besitzen;
Y Wasserstoff oder eine Carbonsäuregruppe oder deren Alkylester, die gegebenenfalls O- oder N-Atome enthalten können, oder Nitril oder die Gruppe -CHR₇OH oder -CH₂NR₈R₉;
darstellen, wobei mindestens einer der Reste X₁ oder X₂ von Wasserstoff verschieden sein muß und es sich im Fall X₁ ungleich X₂, um Gemische verschiedener Isomere bezüglich der Stellung der Substituenten X₁ und X₂ in der 3- oder 4-Position des Pyrrolidinrings, verschiedener Stereoisomere oder um isomerenreine Verbindungen handeln kann.

2. Verwendung nach Anspruch 1, wobei
R₅ und R₆ Wasserstoff;
X₁, X₂ Wasserstoff oder Nitril oder die Gruppen -CH₂OH, -CH₂NH₂ oder -NH₂ oder Carbonsäureester oder -amide, die gegebenenfalls O- oder N-Atomen enthalten,
Y Wasserstoff oder -CH₂OH oder -CH₂NH₂
darstellen.

3. Verwendung nach Anspruch 2, wobei
R₁ eine C₁-C₁₂-Alkyl, C₅-C₆-Cycloalkyl-, Aryl-, Arylalkyl-Gruppe oder gemeinsam mit R₄ eine C₃-C₅-Alkylengruppe;
R₂, R₃ und Y Wasserstoff;
R₄ Wasserstoff oder gemeinsam mit R₁ eine C₃-C₅-Alkylengruppe
darstellen.

4. Verfahren zur Herstellung von gegebenenfalls zellförmigen Kunststoffen nach dem Polyisocyanatpolyadditionsverfahren durch Umsetzung von
a) Polyisocyanaten mit
b) höhermolekularen Verbindungen mit mindestens zwei gegenüber Isocyanaten reaktionsfähigen Wasserstoffatomen, unter Verwendung von
c) Katalysatoren
sowie gegebenenfalls
d) Kettenverlängerungsmitteln und
e) weiteren an sich bekannten Zusatzmitteln wie Treibmitteln, Stabilisatoren und andere,
das dadurch gekennzeichnet ist, daß Katalysatoren gemäß Verwendung nach einem der Ansprüche 1 bis 3 gegebenenfalls in Kombination mit anderen in der Polyurethan-Chemie üblichen Katalysatoren, eingesetzt werden.

## Claims

1. Use of 3,(4)-substituted pyrrolidines corresponding to formula (I) below as catalysts for a polyisocyanate polyaddition process: 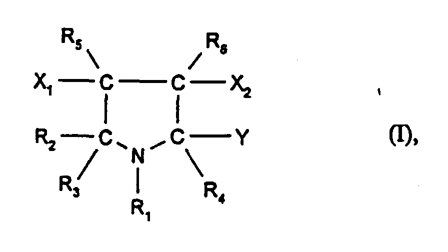 wherein
R₁ denotes hydrogen, or denotes a C₁-C₁₂-alkyl, a C₃-C₇-cycloalkyl or a C₆-C₁₄-aryl or -arylalkyl group, each of which may optionally contain N or O, or R₁ together with R₄ denotes a C₁-C₅-alkylene group or a C₁-C₆-alkylene group which links two pyrrolidine radicals of formula (I) via the N-atom;
R₂ and R₃, independently of one another, denote hydrogen, a C₁-C₁₂-alkyl group, a C₃-C₇-cycloalkyl or -aryl group or a C₆-C₁₄-arylalkyl group, or together denote a C₂-C₉-alkylene group;
R₄ denotes hydrogen, a C₁-C₁₂-alkyl group, a C₃-C₇-cycloalkyl group, a C₆-C₁₄-aryl or -arylalkyl group, or together with R₁ denotes a C₁-C₅-alkylene group;
R₅ and R₆ denote groups having the same meanings as defined for R₂ and R₃, or together form a radical having the following structure: 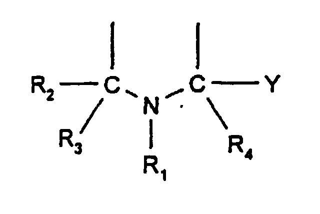
X₁ and X₂, independently of each other (i.e. they may be the same or different), denote hydrogen, carboxylic acid groups or alkyl esters thereof which may optionally contain O or N atoms, or denote nitrile or the groups -CHR₇OH, -CH₂NR₈R₉, -CONR₈R₉, -NR₈R₉ or -CONR₈-(CH₂)₁₋₆-NR₇R₈, wherein
R₇ denotes hydrogen, or a C₁-C₁₂-alkyl, -cycloalkyl, -aryl or -arylalkyl group, and
R₈ and R₉ have the meanings as defined for R₂ and R₃; and
Y denotes hydrogen or a carboxylic acid group or alkyl esters thereof which may optionally contain O or N atoms, or denotes nitrile or the groups -CHR₇OH or -CH₂NR₈R₉,
wherein at least one of the X₁ or X₂ radicals must be different from hydrogen and when X₁ is not the same as X₂ the compounds may be mixtures of different isomers with respect to the position of the X₁ and X₂ substituents in the 3- or 4-position of the pyrrolidine ring, or may be different stereoisomers or isomerically pure compounds.

2. Use according to claim 1, wherein
R₅ and R₆ denote hydrogen;
X₁ and X₂ denote hydrogen or nitrile or the groups -CH₂OH, -CH₂NH₂ or -NH₂, or carboxylic acid esters or amides which optionally contain O or N atoms, and
Y denotes hydrogen, -CH₂OH or -CH₂NH₂.

3. Use according to claim 2, wherein
R₁ denotes a C₁-C₁₂-alkyl group or a C₅-C₆-cycloalkyl, -aryl or -aralkyl group, or together with R₄ denotes a C₃-C₅-alkylene group;
R₂, R₃ and Y denote hydrogen, and
R₄ denotes hydrogen or together with R₁ denotes a C₃-C₅-alkylene group.

4. A process for the production of synthetic materials, which are optionally cellular, by a polyisocyanate polyaddition process, by the reaction of
a) polyisocyanates with
b) higher molecular weight compounds having at least two hydrogen atoms which are capable of reacting with isocyanates, using
c) catalysts
and optionally
d) chain lengthening agents, and
e) other additives known in the art, such as foaming agents, stabilisers and others,
which is characterised in that catalysts are used according to the use claimed in any one of claims 1 to 3, optionally in combination with other catalysts which are customary in polyurethane chemistry.

## Revendications

1. Utilisation, comme catalyseurs pour le procédé de polyaddition de polyisocyanates, de pyrrolidines substituées en 3,(4) correspondant à la formule (I) suivante: 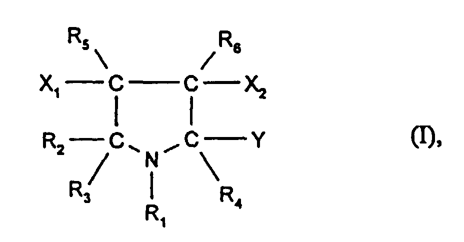 dans laquelle
R₁ représente un atome d'hydrogène, un groupe alkyle on C₁-C₁₂, cycloalkyle en C₃-C₇, aryle ou arylalkyle en C₆-C₁₄, pouvant chacun éventuellement contenir N ou O, ou, on combinaison avec R₄, un groupe alkylène on C₁-C₅, ou un groupe alkylène en C₁-C₆ qui relie deux restes pyrrolidine de formule (I) par l'intermédiaire de l'atome d'azote;
R₂, R₃ représentent indépendamment l'un de l'autre un atome d'hydrogène ou un groupe alkyle en C₁-C₁₂, cycloalkyle en C₃-C₇, aryle ou arylalkyle en C₆-C₁₄, ou forment ensemble un groupe alkylène on C₂-C₉;
R₄ représente un atome d'hydrogène ou un groupe alkyle on C₁-C₁₂, cycloalkyle en C₃-C₇, aryle ou arylalkyle en C₆-C₁₄, ou forme on combinaison avec R₁ un groupe alkylène en C₁-C₅;
R₅, R₆ représentent des groupes ayant la même signification que celle définie pour R₂, R₃, ou forment ensemble un reste de structure 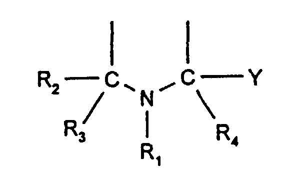
X₁, X₂ représentent, indépendamment l'un de l'autre (c'est-à-dire qu'ils sont identiques ou différents), un atome d'hydrogène, un groupe acide carboxylique ou un de ses esters alkyliques, pouvant éventuellement contenir des atomes de O ou N, un groupe nitrile ou un groupe -CHR₇OH, -CH₂NR₈R₉, -CONR₈R₉, -NR₈R₉ ou -CONR₈-(CH₂)₁₋₆-NR₇R₈, où
R₇ représente un atome d'hydrogène ou un groupe alkyle, cycloalkyle, aryle ou arylalkyle en C₁-C₁₂, et
R₈, R₉ ont la signification définie pour R₂, R₃;
Y représente un atome d'hydrogène ou un groupe acide carboxylique ou un de ses esters alkyliques, pouvant éventuellement contenir des atomes de O ou N, un groupe nitrile ou un groupe -CHR₇OH ou -CH₂NR₈R₉;
au moins l'un des restes X₁ et X₂ devant être différent de l'hydrogène et ces pyrrolidines pouvant être constituées, dans le cas où X₁ est différent de X₂, de mélanges de différents isomères par rapport à la position des substituants X₁ et X₂ en position 3 ou 4 du cycle pyrrolidine, de mélanges de différents stéréoisomères ou de composés isomères purs.

2. Utilisation selon la revendication 1, dans laquelle
R₅ et R₆ représentent l'hydrogène;
X₁, X₂ représentent un atome d'hydrogène, un groupe nitrile ou un groupe -CH₂OH, -CH₂NH₂ ou -NH₂, ou un groupe ester ou amide d'acide carboxylique contenant éventuellement des atomes de O ou N,
Y représente un atome d'hydrogène ou un groupe -CH₂OH ou -CH₂NH₂.

3. Utilisation selon la revendication 2, dans laquelle
R₁ représente un groupe alkyle en C₁-C₁₂, cycloalkyle en C₅-C₆, aryle ou arylalkyle, ou forme en combinaison avec R₄ un groupe alkylène en C₃-C₅;
R₂, R₃ et Y sont des atomes d'hydrogène; et
R₄ représente un atome d'hydrogène ou, en combinaison avec R₁, un groupe alkylène en C₃-C₅.

4. Procédé de préparation de matières plastiques éventuellement cellulaires par le procédé de polyaddition de polyisocyanates, par réaction
a) de polyisocyanates avec
b) des composés de masse molaire élevée contenant au moins deux atomes d'hydrogène réactifs vis-à-vis des isocyanates, en présence
c) de catalyseurs, et éventuellement
d) d'agents d'allongement de chaîne et
e) d'autres additifs connus en soi comme des agents soufflants, des stabilisants et autres,
caractérisé en ce que l'on utilise des catalyseurs correspondant à l'utilisation selon l'une des revendications 1 à 3, éventuellement ai combinaison avec d'autres catalyseurs classiques dans la chimie du polyuréthane.
